Europäisches Patentamt

European Patent Office

Office européen des brevets

(11) Publication number: **0 199 018 B1**

# EUROPEAN PATENT SPECIFICATION

(45) Date of publication of patent specification: **13.01.93**

(51) Int. Cl.⁵: **C07K 15/00**, C12N 15/62

(21) Application number: **86102447.9**

(22) Date of filing: **25.02.86**

(54) **Expression in e.coli of hybrid polypeptides containing the growth hormone releasing factor sequence.**

(30) Priority: **22.03.85 IT 4785685**

(43) Date of publication of application:
**29.10.86 Bulletin 86/44**

(45) Publication of the grant of the patent:
**13.01.93 Bulletin 93/02**

(84) Designated Contracting States:
**AT BE CH DE FR GB LI NL SE**

(56) References cited:
EP-A- 0 068 719     EP-A- 0 108 387
EP-A- 0 129 073     EP-A- 0 160 190
EP-A- 0 206 863     US-A- 4 499 188

NATURE, vol. 300, 18th November 1982, pages 276-278, Macmillan Journals Ltd; J. RIVIER et al.: "Characterization of a growth hormone-releasing factor from a human pancreatic islet tumour"

BIOCHEMISTRY, vol. 20, no. 24, 1981, pages 6997-7004, American Chemical Society; A. FONTANA et al.: "Chemical cleavage of tryptohpanyl and tyrosyl peptide bonds via oxidative halogenation mediated by o-iodosobenzoic acid"

NATURE, vol. 289, 26th February 1981, pages 751-758, Macmillan Journals Ltd; C. YANOFSKY: "Attenuation in the control of expression of bacterial operons"

(73) Proprietor: **ISTITUTO di RICERCA CESARE SERONO SpA**
**Via Valle Caia, 22**
**Ardea (Roma)(IT)**

(72) Inventor: **Canosi, Umberto**
**Via Parco Della Rimembranza**
**Albano(IT)**
Inventor: **de Fazio, Gabriele**
**Via Pio Emanuelli**
**Rome(IT)**
Inventor: **Villa, Stefano**
**Via Oslavia**
**Rome(IT)**
Inventor: **Donini, Silvia**
**L.go degli Orsi**
**Rome(IT)**

(74) Representative: **Kraus, Walter, Dr. et al**
**Patentanwälte Kraus, Weisert & Partner**
**Thomas-Wimmer-Ring 15**
**W-8000 München 22(DE)**

## Description

A group of substances called growth hormone releasing factors (GRF) have recently been isolated from the pancreatic tumor of an acromegalic patient. Guillemin et al, Science 218, 585, 1982; Esch et al, J. Biol. Chem. 258, 1806, 1983.

Several forms of GRF have been purified and their amino acid sequences determined. GRF-44 contains the complete amino acid sequence of GRF-40 and is extended at the carboxyl terminus by four amino acids.

In turn, GRF-40 contains the complete amino acid sequence of GRF-37 and is extended at the carboxyl terminus by three amino acids. The peptide GRF 29 (J. River et al., Nature 300, 276-8, 1982) has also been shown to be biologically active.

Only the carboxyl terminus of GRF-44 (Leu) is amidated (GRF-$NH_2$-44). It is believed that the GRF-$NH_2$-44 is the mature hormone and that GRF-40, 37 and 29 are proteolytic derivatives of it, although all the above mentioned GRFs are biologically active.

It has been reported that GRFs act on both synthesis and releasing of growth hormone by the pituitary gland. Brazeau et al, Proc. Natl. Acad. Sci., 79, 7909, 1982; Baringa et al, Nature, 306, 84, 1983.

It has been suggested that the GRF-44 peptide isolated from the hypothalamus (hhGRF) was identical to that derived from pancreatic tumor (hpGRF) and in fact, immunoreactivity was detected between hhGRF and antibodies raised against hpGRF. Additionally, both peptides gave the same profile when analyzed by HPLC (Bohlen et al, Biochem. Biophys. Res. Commun., 114, 930, 1983). More recently, it was demonstrated that hpGRF and hhGRF have the same amino acid sequence (Ling et al, Proc. Natl. Acad. Sci., 81, 4302, 1984).

The synthesis and characterization of complementary DNA to messenger RNA isolated from the pancreatic tumor that produces hpGRF have demonstrated that the GRF-44 is produced as a pre-prohormone having 107-108 amino acids and the GRF extends from amino acid residue 32 to amino acid residue 75. (Gubler et al, Proc. Natl. Acad. Sci., 80, 4311, 1983; Mayo et al, Nature, 306, 86, 1983).

The Gly-Arg following the GRF-44 sequence resembles a typical amidation site (Boel et al, The EMBO J. 3, 909, 1984; Bradbury et al, Nature, 298, 686, 1982).

The GRFs can be utilized therapeutically in most of the areas now considered candidates for treatment by growth hormone. Examples of such therapeutic uses include treatment of pituitary dwarfism, diabetes determined by abnormal growth hormone production, treatment of wounds and severe burns.

The size of GRF in its several forms is such as to allow its preparation by conventional peptide synthesis methods. Several GRF derivates have indeed been produced by these means and found to be biologically active (Murphy et al, Biochem. Biophys. Res. Commun., 112, 469, 1983; Thorner et al, Lancet, January 1/8, 24, 1983; Adams et al, Lancet, May 14, 1100, 1983; Rosenthal et al, J. Clin. Endocr. Metab., 57, 677, 1983). Furthermore, it possible to synthesize peptides containing an amidated carboxyl at the terminus amino acid. However, production of GRF-44 by chemical means is quite expensive and the production of the peptide by recombinant DNA techniques appears to be more convenient.

European patent application, publication N. 0108387, describes the preparation of synthetic DNA molecules coding for various forms of GRF preceded by a methionine codon which allows, after insertion in an appropriate expression vector, the direct synthesis of a methionylated GRF by an appropriate microorganism. A method is described involving the preparation of two series of oligodeoxyribonucleotide fragments which, when joined in the proper sequence, yield two double stranded DNA molecules coding for the amino and carboxy terminals, respectively, of the GRF peptide.

EP-A-0 160 190 describes new plasmid vectors containing the entire Trp regulatory system including promotor, operator, leader and attenuator, and methods for the expression of hybrid polypeptides in E.coli. The hybrid polypeptides can contain the heterologous sequence of somatostatin.

EP-A-0 129 073 relates to methods and compositions for efficient production of secreted growth hormone releasing factor. The compositions provide for transcription of the DNA sequence encoding the factor in phase with yeast secretory and processing signals for cleavage at the processing signals and removal of the N-terminal polypeptide.

The two double stranded DNA molecules are ligated together to yield the desired GRF structural gene. The preferred expression vectors are derivates of plasmid pBR322 containing the $P_L$ promoter isolated from bacteriophage lambda DNA and inserted between the $tet^R$ and $amp^R$ genes. The presence of the additional methionine amino acid at the N terminus of the GRF molecule raises the possibility of undesired immunological reaction, especially in prolonged therapies.

This invention relates to the production of a GRF by means of a hybrid polypeptide obtained through recombinant DNA techniques and the material used therein.

The hybrid polypeptide contains amino acids 1-323 of TrpE coupled in sequence to the amino acid Trp and the aminoacid sequence of GRF. A non-amidated GRF can be obtained by reduction and carboxyamidomethylation, specific cleavage of the Trp residue followed by gel filtration and purification of GRF by HPLC.

Accordingly, it is the object of this invention to provide a method for the production of GRF as a hybrid polypeptide coded by a plasmid and based on the use of E. Coli Trp promoter/operator followed by Trp leader and attenuator, by the ribosomal binding site of the TrpE gene, by DNA coding for the first two thirds of the TrpE polypeptide, a Trp codon, and by the gene coding for GRF peptide.

Another object of this invention is to provide for the synthesis of a DNA molecule which codes for GRF preceded by a TGG codon for Trp and the nucleotide sequence that allows insertion of this molecule within a plasmid carrying TrpE structural gene while maintaining it in a correct reading frame.

A further object of this invention is to provide a method for growing the microorganism containing the plasmid of the invention to allow the synthesis of a high quantity of the hybrid TrpE-GRF polypeptide.

A still further object of the invention is to provide a method for the extraction of the hybrid polypeptide and for the separation of the desired GRF peptide therefrom through a series of steps.

These and other objects of the invention will be apparent to those skilled in the art from the following detailed description in which:

Fig. 1 shows the nucleotide sequence of the gene coding for GRF-44. The DNA molecule was chemically synthesized by the solid phase phosphotriester method using dinucleotides as building blocks and polystyrene as the solid support. Bgl II, XbaI, BstXI, NarI and BamHI indicate the sites recognized by these restriction endonucleases. Stop indicates the codon for protein synthesis termination;

Fig. 2 is a restriction map of pSP2 plasmid vector in which the thin line represents pBR322 DNA, the thick line represents E. Coli chromosomal DNA carrying the Trp promoter/operator, the Trp leader sequence (TrpL), the entire TrpE structural gene and partial TrpD structural gene (ΔTrpD), Ap$^R$ and Tc$^R$ indicate the genes that confer resistance to ampicillin and tetracycline, respectively, and "Ori" is the origin of replication of this plasmid;

Fig. 3 is a flow chart showing construction of plasmid pSP2del from plasmid pSP2 in which ΔE represents the incomplete TrpE structural gene;

Fig. 4 is a flow chart for the construction of plasmid pSP19 from plasmid pSP2de1; and

Fig. 5 is schematic structure of the amino acid sequence of the hybrid TrpE-GRF polypeptide showing the entire amino acid sequence of the TrpE portion and the first five amino acids of GRF. The entire sequence of GRF-44 is set forth in Fig. 1.

Because of the presence of several restriction enzyme sites on the GRF 44 DNA molecule it is possible to derive another three molecules that constitute a further aspect of this invention, i.e. coding for GRF-40, GRF-37 and GRF-29 peptides.

In fact, the DNA molecule can be degraded with the NarI restriction enzyme and the 3' end fragment can be replaced with the following oligonucleotide:

```
                40

          Ala   Stop

        C GCC   TAG

          GG    ATCCTAG

        NarI      BamHI
```

to generate a DNA molecule coding for GRF-40.

A DNA molecule coding for GRF-37 can be obtained as follows:

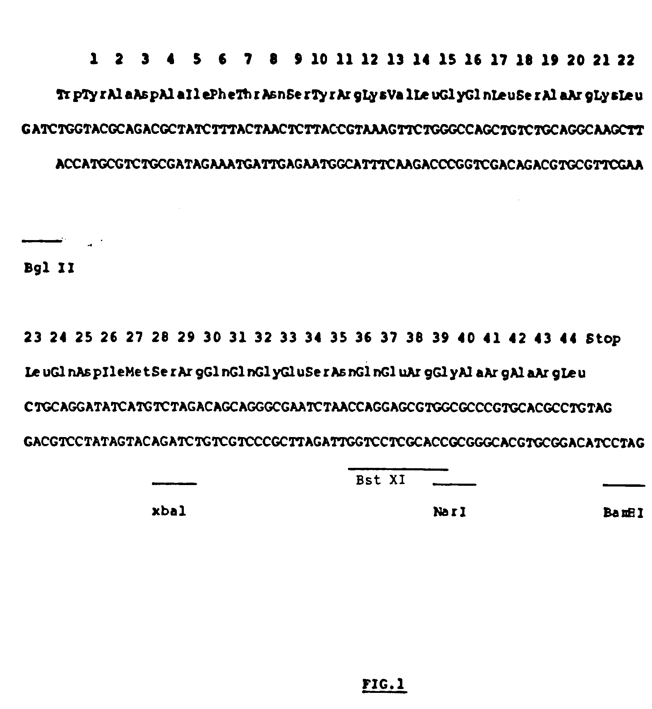

a) The DNA in Fig. 1 is degraded with BstXI restriction enzyme which will generate the following 3' end:

```
              35    36

              Asn   Gln

              ---AAC  CAG  GAGC

              ---TTG  GTC
```

b) The single stranded tail is removed with S1 exonuclease.

c) The following oligonucleotide:

```
              37

              Glu Stop

              GAG TAG

              CTC ATCCTAG
                      BamHI
```

is added to the 3' end to regenerate the 37th codon.

By degrading the DNA molecule in Fig. 1 with XbaI restriction enzyme and substituting the 3' end fragment with the following oligonucleotide:

```
              29

              Arg Stop

              CTAGA TAG

                  T ATCCTAG

              XbaI    BamHI
```

a DNA molecule coding for GRF-29 is obtained.

The construction of the pSP19 plasmid and the preparation of the plasmid derived TrpE-GRF-44 hybrid polypeptide were accomplished as follows.

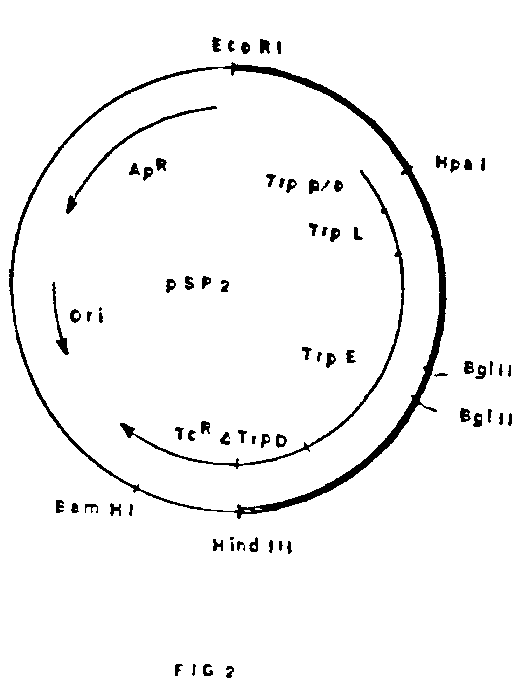

1) Construction of pSP2 plasmid. The pSP2 plasmid was constructed starting from pBR322 (Bolivar et al., Gene, 2, 95-113, 1977) and λED10f (Armstrong et al., Science, 196, 172, 1977 and Helinski et al., in: Recombinant Molecules, Tenth Miles International Symposium, Raven Press, 1977, pgg 151-165), which was used as the source of E. Coli Trp operon DNA, which extends from promoter to TrpD structural gene. pBR322 and λED10f were degraded with EcoRI and HindIII restriction enzymes. The EcoRI-HindIII fragment from λED10f and carrying the Trp operon regulatory functions, the complete TrpE structural gene and the 5' end of the TrpD structural gene were ligated with the HindIII-EcoRI larger fragment of pBR322 by T4 DNA ligase. The ligation mixture was used to transform E. Coli W3110 ΔTrp E5/tna2 cells (Nichols and Yanofsky, Methods in Enzymology, 101, 155, 1983). The transformed cells were plated onto minimal medium plates lacking tryptophane. One Trp + clone was used as source of pSP2 recombinant DNA plasmid. Cells of this clone were grown and stored in rich medium (e.g., NB, Difco) with the addition 50 μg/ml ampicillin (Ap). The restriction map of pSP2 is shown in Fig. 2 where the thick EcoRI-HindIII fragment carries the E.Coli Trp functions and is derived from lambda ED10f, whereas the other DNA is derived from pBR322.

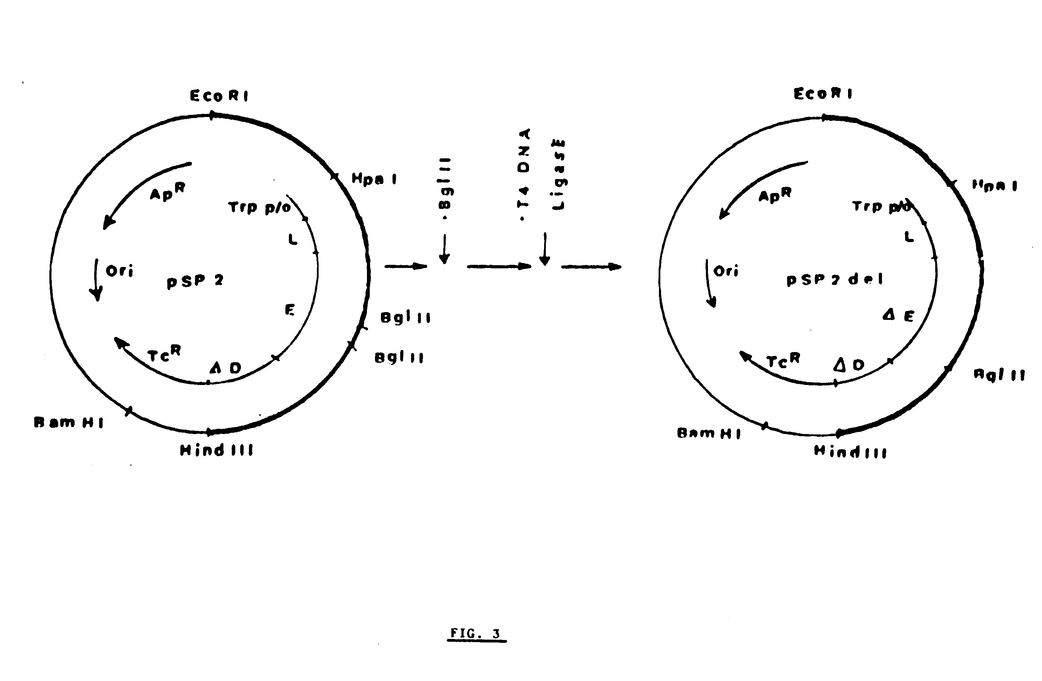

2) Construction of plasmid pSP2del. pSP2 plasmid DNA was degraded with Bgl II endonuclease and the larger fragment was purified by agarose gel electrophoresis and ligated on itself with T4 DNA ligase. The ligation mixture was used to transform W3110 Δ TrpE5/tna 2 cells. Ap^R transformants were selected onto Nutrient Agar (Difco) containing 50 μg/ml Ap. One Ap^R clone was used as source of pSP2del DNA whose restriction map is shown in Fig. 3. (see description of Fig. 2 for details).

The removal of Bgl II fragment from the TrpE structural gene caused the expression of partial TrpE

4

polypeptide, with loss of its enzymatic activity. The pSP2del carrying W3110 Δ TrpE 5tna 2 cells must therefore be grown in the presence of tryptophan. The junction of Bgl II sites in pSP2del creates a new stop codon of the protein synthesis. (Nichols et al, J. Mol. Biol. 146, 45-54, 1981).

The pSP2del derived partial TrpE (Δ TrpE) thus contains 342 aminoacids against the 520 of the whole protein which is coded by pSP2 plasmid (see again Fig. 3).

3) Cloning of Trp-GRF-44 gene pSP2del DNA was degraded with Bgl II and BamHI restriction enzymes and the larger fragment was purified by agarose gel electrophoresis. This DNA was mixed with the synthetic Trp-GRF-44 coding DNA molecule (see Fig. 1) and treated with T4 DNA Ligase.

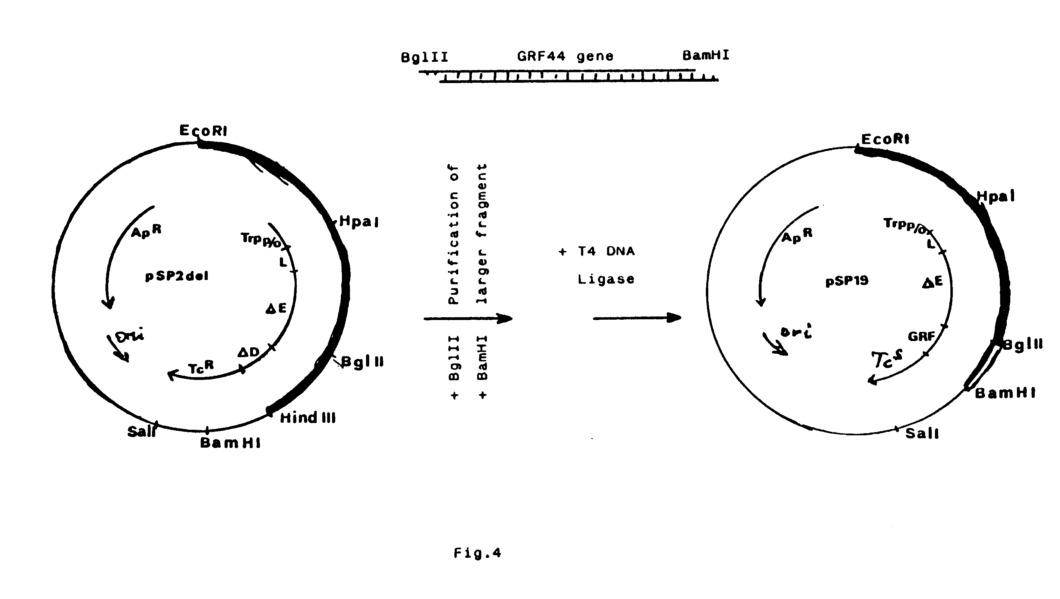

Fig. 4 shows the construction of pSP19 plasmid by insertion of the synthetic gene within the pSP2del plasmid.

$Tc^S$ indicates sensitivity to tetracycline.

The ligation mixture was used to transform W3110 ΔTrpE/tna2 cells and

$Ap^R$ clones were selected on plates containing 50 $\mu$g/ml Ap.

One $Ap^R$ clone which resulted sensitive to tetracycline ($Tc^S$) was used as source of pSP19 DNA.

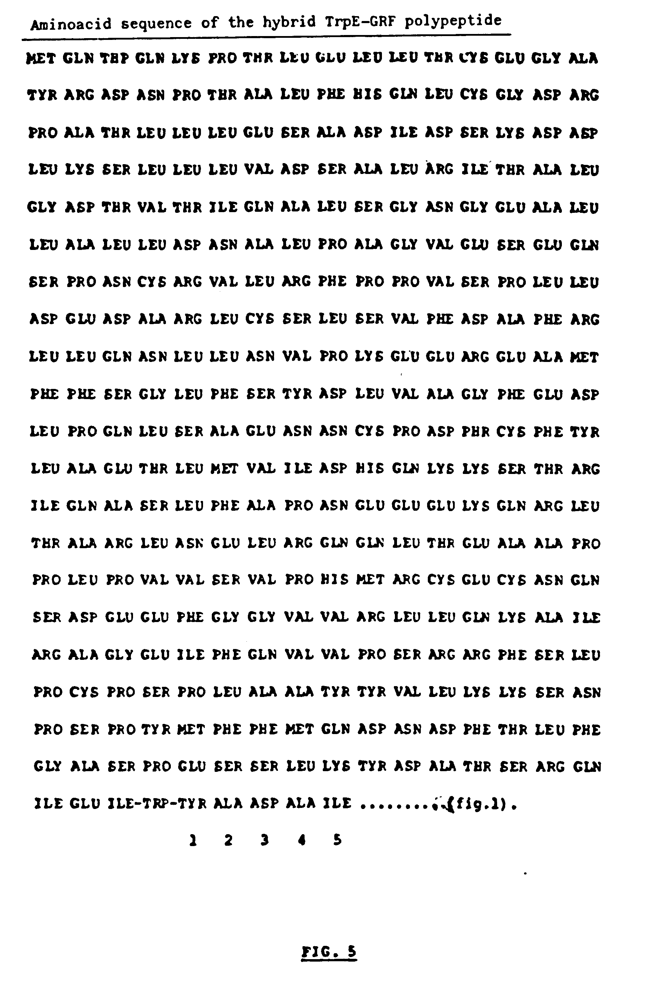

The nucleotide sequence of Trp GRF44 gene allows the synthesis of a hybrid polypeptide between partial TrpE and GRF44 separated by a tryptophane residue. The Trp is degradable by idosobenzoic acid as hereunder described. The hybrid polypeptide coded by pSP19 plasmid DNA has the aminoacid sequence shown in Fig. 5 and is conventionally indicated as TrpE-GRF.

The first 323 aminoacids represent the first two thirds of the TrpE, which are followed by a trp residue and the GRF44 aminoacid sequence. The TrpE-GRF is therefore composed of 368 aminoacids.

Production of the TrpE-GRF-44 hybrid protein

Cells of strain W3110 Δ TrpE5tna2 (pSP19) were grown overnight in 300 ml of SMM (Spizizer Minimal medium) containing per litre of acqueous solution:

| | | |
|---|---|---|
| $(NH_4)_2SO_4$ | 2 | g |
| $KH_2PO_4$ | 6 | g |
| $K_2HPO_4$ | 14 | g |
| $Na.citrate.2H_2O$ | 1 | g |
| $MgSO_4$ | 0.2 | g |

After sterilization by autoclaving the following were added:
10 ml of 40% glucose
3.5 $\mu$g/ml of indole.

The culture (about 4.3 x $10^8$ cells/ml) was diluted in 10 litres of the same medium and the cells were grown under agitation and insufflation of 1 litre of air at 1 Atm pressure per minute.

The composition of the medium and the growth conditions in a 10 litres fermentor have been demonstrated to be ideal to maintain the pSP19 carrying Trp promoter derepressed (thus allowing expression of the TrpE-GRF polypeptide) and also to allow the growth of the cells.

After 22-25 hours of growth the culture reached an OD of about 3.0 at 590 nm. The cells were harvested by centrifugation and stored at -80 C. A sample of these cells was used to analyze the protein content by polyacylamide gel-electrophoresis demonstrating the presence of the desired TrpE-GRF polypeptide.

Purification of TrpE-GRF polypeptide

The frozen cells were thawed in the following buffer: 0.2 M Tris-HCl pH 7.6; 0.2 M NaCl; 0.01 M $CH_3COOMg$; 0.01 M $\beta$-mercaptoethanol and 5% glycerol. The cells were ground in the presence of alumina and the cellular debries were removed by centrifugation.

The acqueous phase was diluted four times with $H_2O$ and the hybrid protein was precipitated by adding 144 g of $(NH_4)_2SO_4$ per liter final solution.

The precipitated proteins were pelletted by centrifugation, dissolved in water and extensively dyalized against 10 mM $NH_4HCO_3$.

The dyalisate was then analyzed by PAGE and lyophilized.

Separation of GRF-44 from the TrpE-GRF hybrid polypeptide

The TrpE-GRF polypeptide was submitted to a series of chemical reactions hereunder described, to allow the separation of GRF peptide moiety.

1) Reduction and carboxyamidomethylation of the Cys residues. The conditions for reduction and carboxyamidomethylation were taken from a paper by G. Allen: Laboratory Techniques in biochemistry and Molecular Biology, Vol. 9, pag.28 eds. T.S. Work and R. H. Burdon, Elsevier, 1981. The TrpE-GRF polypeptide previously prepared and lyophilized was dissolved in the following buffer: Tris-HCl 0.5M; EDTA 01%; Guanidine-HCl 6M pH 8.5.

The final protein concentration was 2%. DTT was then added at a concentration 15 times higher than the Cys content in the protein.

The mixture was then incubated for 2 hours at 50°C and idoacetamide was added at twice DTT concentration. After 30 min of incubation in the dark at room temperature the reaction was interrupted by the addition of beta-mercaptoethanol.

The reaction mixture was then dyalized for 2 hrs against water, and overnight against 10mM $NH_4HCO_3$. This material, containing the carboxyamidomethylated TrpE-GRF polypeptide was then lyophilized.

2) Idosobenzoic acid reaction. The method used was essentially as described by A. Fontana et al.: Biochemistry 20, 6997, 1981. 5 mg of iodosobenzoic acid were dissolved in 375 $\mu$l of 4M Guanidine-HCl, 80% acetic acid. To this solution were added 7.5 $\mu$l of p-cresole and then 5 mg of carboxyamidomethylated TrpE-GRF were dissolved.

The reaction was allowed to proceed for 20 hrs in the dark, at room temperature. 750 $\mu$l of water were then added and after 10 min the mixture was centrifuged for 5 min at 12,000 g. The acqueous phase contains peptides, among which GRF-44.

3) GRF-44 purification The previously obtained acqueous solution was desalted by gel filtration through 1 x 50 cm column of Sephadex G-25, equilibrated against 5% acetic acid. The flow rate was about 3 ml/hr. The excluded material was recovered and its volume reduced by evaporation. The concentrated solution so obtained was analyzed by HPLC using a C18 column equilibrated with 10 mM $H_3PO_4$, brought to pH 3.5 with $Et_3N$.

The peptides were eluted with acetonitrile and collected in fractions that were subsequently analyzed by RIA.

The results demonstrated the presence of immunoactivity in the main peak.

TrpE-GRF40, TrpE-GRF37 and TrpE-GRF29 hybrid polypeptides and the corresponding GRF40, GRF37 and GRF29 peptides can be obtained by procedures analogous to those described above for TrpE-GRF44 and GRF44 production.

W3110 Δ TrpE5 tna2 cells containing the plasmids described here have been deposited at the ATCC and identified as follows:

W3110 Δ TrpE5 tna 2 (pSP2)      ATCC n. 53056
W3110 Δ TrpE5 tna 2 (pSP2-del)   ATCC n. 53058
W3110 Δ TrpE5 tna 2 (pSP19)      ATCC n. 53054

**Claims**

1. A structural gene coding for Trp-GRF wherein the first N-terminal tyrosin codon is immediately preceded by a codon for Trp.

2. A hybrid gene comprising a DNA sequence coding for a TrpE homologous amino acid sequence fused in reading frame to the gene of claim 1.

3. A hybrid gene according to claim 2 in which the TrpE homologous amino acid sequence comprises amino acids 1-323 of TrpE.

4. A hybrid gene according to any of claims 1 to 3 wherein the GRF is selected from GRF-44, GRF-40, GRF-37 and GRF-29.

5. A hybrid gene according to any of claims 1 to 4 operably linked to a DNA sequence capable of

EP 0 199 018 B1

effecting microbial expression of said structural gene.

6. A replicable microbial expression vehicle containing a hybrid gene according to claim 5.

7. A replicable microbial expression vehicle according to claim 6 wherein the vehicle is a plasmid.

8. A microorganism transformed with the expression vehicles according to claims 6 or 7.

9. A microorganism according to claim 8 which is an E. coli strain.

10. A method for the preparation of a hybrid polypeptide comprising a Trp-GRF wherein the first N-terminal amino acid Tyr is immediately preceded by the amino acid Trp characterized in that a microorganism according to any of claims 8 to 9 is grown in a suitable culture medium.

11. A method according to claim 10 wherein the expression vehicle contains a hybrid gene according to any of claims 3 to 5 and the microorganisms are grown in the presence of a concentration of indole sufficient to allow the cells to grow and also to maintain the Trp operon derepressed.

12. A method according to claim 11 wherein the cells are grown with insufflation of an air volume sufficient to partially inactivate indole.

13. A method for recovering GRF from a hybrid polypeptide containing a Trp-GRF wherein the first N-terminal amino acid Tyr is immediately preceded by the amino acid Trp characterized in that the Trp residue joined to the GRF amino acid sequence is cleaved.

14. A method according to claim 13 wherein cleavage is effected by treatment with iodosobenzoic acid.

15. A method according to claim 13 or 14 wherein any Cys residues present in the hybrid polypeptide are reduced and carboxymethylated prior to the Trp cleavage.

16. A method according to any of claims 13 to 15 wherein the GRF is selected from GRF 44, 40, 37 and 29.

17. Hybrid polypeptide containing a Trp-GRF wherein the first N-terminal amino acid Tyr is immediately preceded by the amino acid Trp.

18. Hybrid polypeptide according to claim 17 wherein the Trp-GRF sequence is preceded by a TrpE homologous amino acid sequence.

19. Hybrid polypeptide according to claim 18 wherein the TrpE homologous amino acid sequence is 1-323 of TrpE.

20. Hybrid polypeptide according to any of claims 17 to 19 wherein the GRF sequence is selected from GRF 44, 40, 37 and 29.

**Patentansprüche**

1. Trp-GRF-kodierendes Strukturgen, worin dem ersten N-terminalen Tyrosinkodon unmittelbar ein Trp-Kodon vorausgeht.

2. Hybridgen, umfassend eine DNA-Sequenz, die eine dem TrpE homologe Aminosäuresequenz kodiert, fusioniert im Leseraster an das Gen nach Anspruch 1.

3. Hybridgen nach Anspruch 2, **dadurch gekennzeichnet,** daß die dem TrpE homologe Aminosäuresequenz die Aminosäuren 1 bis 323 von TrpE enthält.

4. Hybridgen nach einem der Ansprüche 1 bis 3, **dadurch gekennzeichnet,** daß der GRF aus GRF-44, GRF-40, GRF-37 und GRF-29 ausgewählt ist.

7

**5.** Hybridgen nach einem der Ansprüche 1 bis 4, funktionell gebunden an eine DNA-Sequenz, die die mikrobielle Expression des Strukturgens bewirken kann.

**6.** Replizierbares, mikrobielles Expressionsvehikel, enthaltend ein Hybridgen nach Anspruch 5.

**7.** Replizierbares, mikrobielles Expressionsvehikel nach Anspruch 6, **dadurch gekennzeichnet,** daß das Vehikel ein Plasmid ist.

**8.** Mikroorganismus, transformiert mit den Expressionsvehikeln nach den Ansprüchen 6 oder 7.

**9.** Mikroorganismus nach Anspruch 8, **dadurch gekennzeichnet,** daß er ein E. coli-Stamm ist.

**10.** Verfahren zur Herstellung eines hybriden Polypeptids, umfassend einer Trp-GRF, worin der ersten N-terminalen Aminosäure Tyr unmittelbar die Aminosäure Trp vorausgeht, **dadurch gekennzeichnet,** daß ein Mikroorganismus nach einem der Ansprüche 8 bis 9 in einem geeigneten Kulturmedium gezüchtet wird.

**11.** Verfahren nach Anspruch 10, **dadurch gekennzeichnet,** daß das Expressionsvehikel ein hybrides Gen nach einem der Ansprüche 3 bis 5 enthält und die Mikroorganismen in Gegenwart einer Indolkonzentration, die ausreichend ist, um die Zellen wachsen zu lassen und auch um das Trp-Operon dereprimiert zu halten, gezüchtet werden.

**12.** Verfahren nach Anspruch 11, **dadurch gekennzeichnet,** daß die Zellen unter Belüftung mit einem Luftvolumen, das ausreichend ist, um Indol teilweise zu inaktivieren, gezüchtet werden.

**13.** Verfahren zur Gewinnung von GRF aus einem hybriden Polypeptid, das ein Trp-GRF, worin der ersten N-terminalen Aminosäure Tyr unmittelbar die Aminosäure Trp vorausgeht, enthält, **dadurch gekennzeichnet,** daß der Trp-Rest, der an die GRF-Aminosäuresequenz angefügt ist, abgespalten wird.

**14.** Verfahren nach Anspruch 13, **dadurch gekennzeichnet,** daß die Spaltung durch Behandlung mit Iodosobenzoesäure durchgeführt wird.

**15.** Verfahren nach Anspruch 13 oder 14, **dadurch gekennzeichnet,** daß irgendwelche Cys-Reste, die in dem hybriden Polypeptid vorhanden sind, vor der Trp-Spaltung reduziert und carboxymethyliert werden.

**16.** Verfahren nach einem der Ansprüche 13 bis 15, **dadurch gekennzeichnet,** daß der GRF aus GRF 44, 40, 37 und 29 ausgewählt wird.

**17.** Hybrides Polypeptid, enthaltend einen Trp-GRF, worin der ersten N-terminalen Aminosäure Tyr unmittelbar die Aminosäure Trp vorausgeht.

**18.** Hybrides Polypeptid nach Anspruch 17, **dadurch gekennzeichnet,** daß der Trp-GRF-Sequenz eine dem TrpE homologe Aminosäuresequenz vorausgeht.

**19.** Hybrides Polypeptid nach Anspruch 18, **dadurch gekennzeichnet,** daß die dem TrpE homologe Aminosäuresequenz 1 bis 323 von TrpE ist.

**20.** Hybrides Polypeptid nach einem der Ansprüche 17 bis 19, **dadurch gekennzeichnet,** daß die GRF-Sequenz aus GRF 44, 40, 37 und 29 ausgewählt ist.

**Revendications**

**1.** Un gène de structure codant pour Trp-GRF dans lequel le premier codon de tyrosine N-terminale est immédiatement précédé parm un codon pour Trp.

**2.** Un gène hybride comprenant une séquence d'ADN codant pour une séquence d'aminoacides homologue TrpE fusionnée en phase de lecture au gène selon la revendication 1.

3. Un gène hybride selon la revendication 2, dans lequel la séquence d'aminoacides homologue TrpE comprend les aminoacides 1-323 de TrpE.

4. Un gène hybride selon l'une quelconque des revendications 1 à 3, dans lequel le GRF est choisi parmi GRF-44, GRF-40, GRF-37 et GRF-29..

5. Un gène hybride selon l'une quelconque des revendications 1 à 4 lié fonctionnellement à une séquence d'ADN capable d'effectuer l'expression microbienne dudit gène de structure.

6. Un véhicule d'expression microbienne réplicable contenant un gène hybride selon la revendication 5.

7. Un véhicule d'expression microbienne réplicable selon la revendication 6,dans lequel le véhicule est un plasmide.

8. Un microorganisme transformé par les véhicules d'expression selon les revendications 6 et 7.

9. Un microorganisme selon la revendication 8 qui est une souche E. Coli.

10. Un procédé pour la préparation d'un polypeptide hybride comprenant un Trp-GRF dans lequel le premier aminoacide Tyr N-terminal est immédiatement précédé par l'aminoacide Trp, caractérisé en ce qu'un microorganisme selon la revendication 8 ou 9 est cultivé dans un milieu de culture convenable.

11. Un procédé selon la revendication 10, dans lequel le véhicule d'expression contient un gène hybride selon l'une quelconque des revendications 3 à 5 et les microorganismes sont cultivés en présence d'une concentration suffisante d'indole pour permettre la croissance des cellules et également pour maintenir l'opéron Trp déréprimé.

12. Un procédé selon la revendication 11, dans lequel les cellules sont cultivées avec injection d'un volume d'air suffisant pour inactiver partiellement l'indole.

13. Un procédé pour récupérer le GRF d'un polypeptide contenant un Trp-GRF dans lequel le premier aminoacide N-terminal Tyr est immédiatement précédé par l'aminoacide Trp, caractérisé en ce que le résidu Trp relié à la séquence d'aminoacides GRF est coupé.

14. Un procédé selon la revendication 13, dans lequel la coupure est effectuée par traitement par l'acide iodosobenzoïque.

15. Un procédé selon la revendication 13 ou 14, dans lequel n'importe lesquels des résidus Cys présents dans le polypeptide hybride sont réduits et carboxyméthylés avant la coupure de Trp.

16. Un procédé selon l'une quelconque des revendications 13 à 15, dans lequel le GRF est choisi parmi GRF-44,40,37 et 29.

17. Polypeptide hybride contenant un Trp-GRF dans lequel le premier aminoacide N-terminal Tyr est immédiatement précédé par l'aminoacide Trp.

18. Polypeptide hybride selon la revendication 17, dans lequel la séquence Trp-GRF est précédée par une séquence d'aminoacides homologue TrpE.

19. Polypeptide hybride selon la revendication 18, dans lequel la séquence d'aminoacides homologue TrpE est 1-323 de TrpE.

20. Polypeptide hybride selon l'une quelconque des revendications 17 à 19, dans lequel la séquence GRE est choisie parmi GRF-44,40,37 et 29.

```
       1   2   3   4   5   6   7   8   9  10  11  12  13  14  15  16  17  18  19  20  21  22

     TrpTyrAlaAspAlaIlePheThrAsnSerTyrArgLysValLeuGlyGlnLeuSerAlaArgLysLeu

   GATCTGGTACGCAGACGCTATCTTTACTAACTCTTACCGTAAAGTTCTGGGCCAGCTGTCTGCAGGCAAGCTT

     ACCATGCGTCTGCGATAGAAATGATTGAGAATGGCATTTCAAGACCCGGTCGACAGACGTGCGTTCGAA
```

——— · ·

Bgl II

```
      23  24  25  26  27  28  29  30  31  32  33  34  35  36  37  38  39  40  41  42  43  44  Stop

     LeuGlnAspIleMetSerArgGlnGlnGlyGluSerAsnGlnGluArgGlyAlaArgAlaArgLeu

   CTGCAGGATATCATGTCTAGACAGCAGGGCGAATCTAACCAGGAGCGTGGCGCCCGTGCACGCCTGTAG

   GACGTCCTATAGTACAGATCTGTCGTCCCGCTTAGATTGGTCCTCGCACCGCGGGCACGTGCGGACATCCTAG
```

———            Bst XI    ———                    ———

xbal                              NarI                         BamHI


FIG.1

FIG 2

FIG. 3

EP 0 199 018 B1

Fig.4

13

Aminoacid sequence of the hybrid TrpE-GRF polypeptide

MET GLN THP GLN LYS PRO THR LEU GLU LEU LEU THR CYS GLU GLY ALA

TYR ARG ASP ASN PRO THR ALA LEU PHE HIS GLN LEU CYS GLY ASP ARG

PRO ALA THR LEU LEU LEU GLU SER ALA ASP ILE ASP SER LYS ASP ASP

LEU LYS SER LEU LEU LEU VAL ASP SER ALA LEU ARG ILE THR ALA LEU

GLY ASP THR VAL THR ILE GLN ALA LEU SER GLY ASN GLY GLU ALA LEU

LEU ALA LEU LEU ASP ASN ALA LEU PRO ALA GLY VAL GLU SER GLU GLN

SER PRO ASN CYS ARG VAL LEU ARG PHE PRO PRO VAL SER PRO LEU LEU

ASP GLU ASP ALA ARG LEU CYS SER LEU SER VAL PHE ASP ALA PHE ARG

LEU LEU GLN ASN LEU LEU ASN VAL PRO LYS GLU GLU ARG GLU ALA MET

PHE PHE SER GLY LEU PHE SER TYR ASP LEU VAL ALA GLY PHE GLU ASP

LEU PRO GLN LEU SER ALA GLU ASN ASN CYS PRO ASP THR CYS PHE TYR

LEU ALA GLU THR LEU MET VAL ILE ASP HIS GLN LYS LYS SER THR ARG

ILE GLN ALA SER LEU PHE ALA PRO ASN GLU GLU GLU LYS GLN ARG LEU

THR ALA ARG LEU ASN GLU LEU ARG GLN GLN LEU THR GLU ALA ALA PRO

PRO LEU PRO VAL VAL SER VAL PRO HIS MET ARG CYS GLU CYS ASN GLN

SER ASP GLU GLU PHE GLY GLY VAL VAL ARG LEU LEU GLN LYS ALA ILE

ARG ALA GLY GLU ILE PHE GLN VAL VAL PRO SER ARG ARG PHE SER LEU

PRO CYS PRO SER PRO LEU ALA ALA TYR TYR VAL LEU LYS LYS SER ASN

PRO SER PRO TYR MET PHE PHE MET GLN ASP ASN ASP PHE THR LEU PHE

GLY ALA SER PRO GLU SER SER LEU LYS TYR ASP ALA THR SER ARG GLN

ILE GLU ILE-TRP-TYR ALA ASP ALA ILE ........,.(fig.1).

1   2   3   4   5

FIG. 5